# EUROPEAN PATENT APPLICATION

(11) **EP 4 261 188 A1**
(43) Date of publication of application: **18.10.2023**
(21) Application number: 21903446.9
(22) Date of filing: 08.12.2021
(51) Int. Cl.: C01B 33/18, C01B 37/00, A61Q 5/02, A61Q 5/04, A61Q 5/06, A61Q 5/12, A61K 8/25, A61K 8/81, A61L 9/014, A61L 9/16, B01J 20/10, B01J 20/32

(54) **SURFACE-MODIFIED AND METAL-DOPED POROUS SILICA**

(30) Priority: 09.12.2020 JP 2020204555
(71) Applicant: Toyo Seikan Group Holdings, Ltd., Shinagawa-ku Tokyo 141-8627 (JP)
(72) Inventor: OHASHI, Kazuaki, Yokohama-shi, Kanagawa 240-0062 (JP); KIMURA, Mariko, Yokohama-shi, Kanagawa 240-0062 (JP); IKUTAME, Daisuke, Yokohama-shi, Kanagawa 240-0062 (JP)
(74) Representative: Körfer, Thomas
(86) International application number: PCT/JP2021/045186
(87) International publication number: WO 2022/124347

(57) **Abstract**

An object of the present invention is to provide a metal-doped porous silica that can be blended into an article, for example, a cosmetic product such as a perm treatment agent, and kept stably dispersed therein. The metal-doped porous silica of the present invention as a means for resolution is surface-modified with a vinylpyrrolidone unit-containing polymer. As specific examples of vinylpyrrolidone unit-containing polymers, a copolymer of vinylpyrrolidone and dimethylaminoethyl methacrylate, polyvinylpyrrolidone, and the like can be mentioned.

## Description

### Technical Field

The present invention relates to a surface-modified, metal-doped porous silica.

### Background Art

As is well known, porous silica has been used as an adsorbent, a humidity agent, a catalyst carrier, or the like in various fields. In recent years, various attempts have been made to increase the functionality of porous silica. As a research result, the present inventors have also reported, in Patent Document 1, that porous silica doped with a metal such as copper has an excellent deodorizing effect on sulfur-containing odors.

The metal-doped porous silica reported by the present inventors in Patent Document 1 is expected to be utilized, for example, as a deodorizing material for sulfur-containing odors remaining in the hair after a perm performed using a sulfur-containing substance such as cysteamine, L-cysteine, or thioglycolic acid as a reducing agent. However, in order to make full use of such an effect, how the metal-doped porous silica is blended into a perm treatment agent and kept stably dispersed therein is important. In addition, also in the case where the metal-doped porous silica is blended into an article other than perm treatment agents, similarly, the blended silica needs to be kept stably dispersed.

### Prior Art Documents

### Patent Document

Patent Document 1: JP2020-15640A

### Summary of the Invention

### Problems that the Invention is to Solve

Thus, an object of the present invention is to provide a metal-doped porous silica that can be blended into an article, for example, a cosmetic product such as a perm treatment agent, and kept stably dispersed therein.

### Means for Solving the Problems

The present inventors have conducted extensive research in view of the above points. As a result, they have found that when a metal-doped porous silica is added as it is to an aqueous solution or aqueous dispersion of a cationic polymer such as polyquaternium-10 (quaternary ammonium salt of hydroxyethylcellulose with glycidyltrimethylammonium chloride), polyquaternium-11 (quaternary ammonium salt of a copolymer of vinylpyrrolidone and dimethylaminoethyl methacrylate with diethyl sulfate), or amodimethicone, or of a nonionic polymer polyvinylpyrrolidone, which have widely been used as an ingredient for cosmetic products such as perm treatment agents, etc., the metal-doped porous silica is not kept stably dispersed, forming precipitates, and also that the formation of precipitates can be suppressed by surface-modifying the metal-doped porous silica with a vinylpyrrolidone unit-containing polymer.

The metal-doped porous silica of the present invention accomplished based on the above findings is, as defined in claim 1, surface-modified with a vinylpyrrolidone unit-containing polymer.

In addition, the metal-doped porous silica according to claim 2 is the metal-doped porous silica according to claim 1, wherein the metal doped in the porous silica is at least one member selected from the group consisting of copper, aluminum, zirconium, cobalt, manganese, and iron.

In addition, the metal-doped porous silica according to claim 3 is the metal-doped porous silica according to claim 2, wherein the metal doped in the porous silica is copper and/or aluminum.

In addition, the metal-doped porous silica according to claim 4 is the metal-doped porous silica according to claim 1, wherein the vinylpyrrolidone unit-containing polymer is a copolymer of a vinylpyrrolidone unit and a non-vinylpyrrolidone unit.

In addition, the metal-doped porous silica according to claim 5 is the metal-doped porous silica according to claim 4, wherein the copolymer of a vinylpyrrolidone unit and a non-vinylpyrrolidone unit is a copolymer of vinylpyrrolidone and dimethylaminoethyl methacrylate.

In addition, the metal-doped porous silica according to claim 6 is the metal-doped porous silica according to claim 1, wherein the vinylpyrrolidone unit-containing polymer is polyvinylpyrrolidone.

In addition, the method for producing a metal-doped porous silica surface-modified with a vinylpyrrolidone unit-containing polymer of the present invention comprises, as defined in claim 7, a step in which a slurry of a metal-doped porous silica suspended in a dispersion medium is housed in a treatment container together with a vinylpyrrolidone unit-containing polymer and balls for use in ball milling (media) (a dispersion medium may further be housed), and the treatment container housing them is put on a ball mill stand and rotated, thereby surface-treating the metal-doped porous silica.

In addition, the slurry of the present invention comprises, as defined in claim 8, a metal-doped porous silica surface-modified with a vinylpyrrolidone unit-containing polymer suspended in a dispersion medium.

In addition, the present invention is, as defined in claim 9, use of the metal-doped porous silica surface-modified with a vinylpyrrolidone unit-containing polymer according to claim 1 for deodorizing an article by blending into an article containing at least one member selected from the group consisting of polyquaternium-10, polyquaternium-11, amodimethicone, and polyvinylpyrrolidone.

### Effect of the Invention

According to the present invention, a metal-doped porous silica that can be blended into an article, for example, a cosmetic product such as a perm treatment agent, and kept stably dispersed therein can be provided.

### Mode for Carrying Out the Invention

The metal-doped porous silica of the present invention is surface-modified with a vinylpyrrolidone unit-containing polymer.

In the present invention, the metal-doped porous silica may be the one described by the present inventors in JP2020-15640A, for example. Here, "metal-doped porous silica" means a porous silica in which a metal is incorporated through chemical bonding into an inorganic network composed of porous silica-forming siloxane bonds, and is specifically as follows.

As metals doped in the porous silica, for example, copper, aluminum, zirconium, cobalt, manganese, and iron can be mentioned. They may be used alone or as a combination of two or more kinds.

The metal content in the metal-doped porous silica (in the case of using a combination of two or more kinds of metals, their total amount) is, for example, 0.01 to 10 wt%, preferably 0.1 to 5 wt%. When the metal content in the metal-doped porous silica is less than 0.01 wt%, it may be impossible to obtain a sufficient deodorizing effect, while a porous silica doped with more than 10 wt% of a metal may be difficult to produce. In the case where a combination of two or more kinds of metals is used, the content ratio between the metals may be such that the content of one metal is 0.1 to 2 times the content of the other metal, for example.

As a porous silica, for example, a mesoporous silica in which fine pores (mesopores) having a diameter of 2 to 50 nm are regularly arranged can be mentioned.

It is preferable that the porous silica has a specific surface area of 500 to 2000 m²/g, for example, for the reason that durability can be maintained.

The production of a metal-doped mesoporous silica can be performed according to the following method known per se described in JP2020-15640A, for example.

### (Step 1)

First, a surfactant and a raw material for doping a metal in a mesoporous silica are dissolved in a solvent and stirred at 30 to 200°C for 0.5 to 10 hours, for example, thereby forming micelles of the surfactant.

The amount of surfactant dissolved in the solvent is, for example, 10 to 400 mmol/L, preferably 50 to 150 mmol/L. Alternatively, the amount of surfactant dissolved in the solvent is, per 1 mol of a silica raw material added in the below-described Step 2, for example, 0.01 to 5.0 mol, preferably 0.05 to 1.0 mol.

As the surfactant, any of cationic surfactants, anionic surfactants, and nonionic surfactants may be used, but cationic surfactants such as alkylammonium salts are preferable. As alkylammonium salts, those having a Cs or higher alkyl group are preferable, and, in view of industrial availability, those having a C₁₂₋₁₈ alkyl group are more preferable. As specific examples of alkylammonium salts, hexadecyltrimethylammonium chloride, cetyltrimethylammonium bromide, stearyltrimethylammonium bromide, cetyltrimethylammonium chloride, stearyltrimethylammonium chloride, dodecyltrimethylammonium bromide, octadecyltrimethylammonium bromide, dodecyltrimethylammonium chloride, octadecyltrimethylammonium chloride, didodecyldimethylammonium bromide, ditetradecyldimethylammonium bromide, didodecyldimethylammonium chloride, ditetradecyldimethylammonium chloride, and the like can be mentioned. Surfactants may be used alone or as a combination of two or more kinds.

The amount of raw material for doping a metal in a mesoporous silica (in the case of using a combination of two or more kinds of metals, the total amount of all the raw materials) dissolved in the solvent is, per 1 mol of a silica raw material added in the below-described Step 2, for example, 0.001 to 0.5 mol, preferably 0.01 to 0.1 mol.

As the raw material for doping a metal in a mesoporous silica, for example, a metal nitrate, sulfate, chloride, or oxychloride can be used. In the case of doping with copper, it is preferable to use copper nitrate or copper chloride. In the case of doping with aluminum, it is preferable to use aluminum chloride. In the case of doping with zirconium, it is preferable to use zirconium oxychloride. In the case of doping with cobalt, it is preferable to use cobalt nitrate. In the case of doping with manganese, it is preferable to use manganese chloride. In the case of doping with iron, it is preferable to use iron chloride. Raw materials for doping a metal may be used alone or as a combination of two or more kinds.

As the solvent, for example, water can be used. The solvent may be a mixed solvent of water and a water-soluble organic solvent such as methanol, ethanol, and a polyhydric alcohol including diethylene glycol, glycerin, or a like.

### (Step 2)

Next, in the solution where the surfactant forms micelles obtained in Step 1, a silica raw material is dissolved at room temperature, for example, and stirred until uniform to allow the silica raw material to accumulate on the surface of the surfactant micelles. The amount of silica raw material dissolved in the solution is 0.2 to 1.8 mol/L, for example. Alternatively, in the case where water or a mixed solvent of water and a water-soluble organic solvent is used as the solvent, the amount is 0.001 to 0.05 mol per 1 mol of water, for example.

The silica raw material is not particularly limited as long as it forms an inorganic network composed of mesoporous silica-forming siloxane bonds upon a dehydration condensation. As specific examples of silica raw materials, tetraalkoxysilanes, such as tetraethoxysilane, tetramethoxysilane, and tetra-n-butoxysilane, and sodium silicate can be mentioned. Tetraalkoxysilanes are preferable, and tetraethoxysilane is more preferable. Silica raw materials may be used alone or as a combination of two or more kinds.

### (Step 3)

Next, the silica raw material accumulated on the surface of the surfactant micelles is subjected to a dehydration condensation to form an inorganic network composed of mesoporous silica-forming siloxane bonds, and, at the same time, a metal is incorporated through chemical bonding into the inorganic network. The dehydration condensation of the silica raw material can be caused, for example, by adding a basic aqueous solution into the system to raise the pH, followed by stirring at room temperature for 1 hour or more. The basic aqueous solution is preferably added such that the pH immediately after the addition is 8 to 14, more preferably 9 to 11. As specific examples of basic aqueous solutions, an aqueous sodium hydroxide solution, an aqueous sodium carbonate solution, and aqueous ammonia can be mentioned, and an aqueous sodium hydroxide solution is preferable. Basic aqueous solutions may be used alone or as a combination of two or more kinds. Incidentally, the dehydration condensation of the silica raw material can also be caused by adding an acidic aqueous solution such as an aqueous hydrochloric acid solution into the system to lower the pH, followed by stirring.

### (Step 4)

Finally, the surfactant micelles having formed on the surface thereof an inorganic network composed of mesoporous silica-forming siloxane bonds, into which a metal has been incorporated through chemical bonding, obtained in Step 3 are collected by filtration as precipitates, dried at 30 to 70°C for 10 to 48 hours, for example, and then calcined at 400 to 600°C for 1 to 10 hours to obtain the intended metal-doped mesoporous silica. The metal-doped mesoporous silica thus obtained may be pulverized in a mixer or mill as necessary to have the desired particle size (e. g., a median diameter of 0.01 to 100 µm is preferable for the reason that such a silica can be easily kept stably dispersed in a perm treatment agent).

Incidentally, the addition of the raw material for doping a metal in a mesoporous silica into the system is not limited to the embodiment in which the raw material is dissolved in a solvent together with a surfactant in the above Step 1, and may also be an embodiment in which the raw material is dissolved in the solution in Step 2 or Step 3 as long as it takes place before the formation of an inorganic network composed of mesoporous silica-forming siloxane bonds upon the dehydration condensation of the silica raw material in Step 3 is completed.

In the present invention, what is used to surfacemodify the metal-doped porous silica is a vinylpyrrolidone unit-containing polymer. The vinylpyrrolidone unit-containing polymer may be a copolymer of a vinylpyrrolidone unit and a non-vinylpyrrolidone unit, for example. As specific examples thereof, a copolymer of vinylpyrrolidone and dimethylaminoethyl methacrylate, a copolymer of vinylpyrrolidone and methylvinylimidazolinium chloride, a copolymer of vinylpyrrolidone and dimethylaminopropylamide methacrylate, a copolymer of vinylpyrrolidone and quaternized imidazoline, a copolymer of vinylpyrrolidone, vinylcaprolactam, and methylvinylimidazolium methylsulfate, and the like can be mentioned. These copolymers are convenient in that their quaternary ammonium salts have already been used as raw materials for cosmetic products under the INCI names of polyquaternium-11, polyquaternium-16, polyquaternium-28, polyquaternium-44, and polyquaternium-46, respectively. In addition, as copolymers of a vinylpyrrolidone unit and a non-vinylpyrrolidone unit, a copolymer of vinylpyrrolidone and vinyl acetate, a copolymer of vinylpyrrolidone and eicosene, a copolymer of vinylpyrrolidone and hexadecene, a copolymer of vinylpyrrolidone and styrene, a copolymer of vinylpyrrolidone and dimethylaminoethyl methacrylate, a copolymer of vinylpyrrolidone, vinylcaprolactam, and dimethylaminoethyl methacrylate, and the like can also be used. They have also already been used as raw materials for cosmetic products and are convenient in this respect. The vinylpyrrolidone unit-containing polymer may be polyvinylpyrrolidone. Polyvinylpyrrolidone has also already been used as a raw material for cosmetic products and is convenient in this respect. In view of an adhesion property to the metal-doped porous silica, an ease of a surface modification, and the like, the favorable molecular weight of the vinylpyrrolidone unit-containing polymer is, depending on its kind, within a range of 5000 to 5000000, for example. In the case where the vinylpyrrolidone unit-containing polymer is a copolymer of vinylpyrrolidone and dimethylaminoethyl methacrylate, its molecular weight is favorably within a range of 100000 to 1200000, while in the case of polyvinylpyrrolidone, its molecular weight is favorably within a range of 40000 to 1600000. In addition, the favorable glass transition temperature (Tg) for the vinylpyrrolidone unit-containing polymer is, depending on its kind, within a range of 120 to 200°C, for example.

The method for surface-modifying a metal-doped porous silica with a vinylpyrrolidone unit-containing polymer is not particularly limited, and may be performed by mixing a metal-doped porous silica and a vinylpyrrolidone unit-containing polymer after adjusting the temperature as necessary, and stirring. However, as a favorable method, a method in which a slurry of a metal-doped porous silica suspended in a dispersion medium is housed in a treatment container together with a vinylpyrrolidone unit-containing polymer and balls for use in ball milling (media) (a dispersion medium may further be housed), and the treatment container housing them is put on a ball mill stand and rotated (the rotation speed is selected within a range of 15 to 500 rpm, for example), thereby surface-treating the metal-doped porous silica, can be mentioned. According to this method, a metal-doped porous silica surface-modified with a vinylpyrrolidone unit-containing polymer can be easily obtained in the form of being contained in a slurry having excellent dispersibility in a perm treatment agent containing a cationic polymer such as polyquaternium-10, polyquaternium-11, or amodimethicone, or a nonionic polymer polyvinylpyrrolidone. The time of ball milling is, for example, 1 to 50 hours, preferably 6 to 30 hours. As the dispersion medium in the slurry of the metal-doped porous silica suspended therein, or as the dispersion medium that may further be housed in the treatment container, water can be used, for example. Water used as a dispersion medium may contain a water-soluble organic solvent such as methanol, ethanol, and a polyhydric alcohol including diethylene glycol, glycerin, or a like, but the water content is preferably 50 wt% or more. The pH of the dispersion medium is, for example 5 to 11, preferably 6 to 9. When the pH of the dispersion medium is less than 5, the metal doped in the porous silica may dissolve, while when the pH of the dispersion medium is more than 11, the porous silica may dissolve. In addition, when the pH of the dispersion medium is too acidic or too alkaline, the properties of a perm treatment agent may be adversely affected.

With respect to the amounts of metal-doped porous silica and vinylpyrrolidone unit-containing polymer used, it is preferable that the weight of the polymer is 0.1 times or more the weight of the porous silica. When the weight of the vinylpyrrolidone unit-containing polymer is too small relative to the weight of the metal-doped porous silica, the effect of surface-modifying the porous silica with the polymer may not be sufficiently obtained, resulting in reduced dispersibility in a perm treatment agent. When the weight of the vinylpyrrolidone unit-containing polymer is up to 0.5 times the weight of the metal-doped porous silica, almost the entire amount or the entire amount of the polymer can adhere to the porous silica, and the effect of surface-modifying the porous silica with the polymer can be sufficiently obtained. When the weight of the vinylpyrrolidone unit-containing polymer is more than 0.5 times the weight of the metal-doped porous silica, the amount of free polymer not adhering to the porous silica contained in the slurry increases, but there is no particular problem if the polymer is a material that has already been used as a raw material for cosmetic products. However, it is preferable that the upper limit of the weight of the vinylpyrrolidone unit-containing polymer is twice the weight of the metal-doped porous silica. A slurry containing a large amount of free vinylpyrrolidone unit-containing polymer is highly viscous and difficult to handle. In addition to this, when such a slurry is added to a perm treatment agent, the composition of the perm treatment agent may be affected. Incidentally, the content of the metal-doped porous silica surface-modified with a vinylpyrrolidone unit-containing polymer in the slurry is preferably 0.1 to 10 wt%, for example, in terms of the handleability of the slurry and the like. With respect to balls for use in ball milling (e. g., 1 to 5 mmφ alumina balls or zirconia balls), the number of balls used is preferably such that the weight thereof is 1 to 5 times the total weight of the metal-doped porous silica, the vinylpyrrolidone unit-containing polymer, and the dispersion medium.

The perm treatment agent into which the metal-doped porous silica surface-modified with a vinylpyrrolidone unit-containing polymer is blended may be for straight perm treatment or permanent wave treatment. In addition, the perm treatment agent into which the metal-doped porous silica surface-modified with a vinylpyrrolidone unit-containing polymer is blended may be a first agent containing a reducing agent such as cysteamine, L-cysteine, or thioglycolic acid, a second agent containing an oxidizing agent such as hydrogen peroxide solution or bromate, or an intermediate treatment agent or post-treatment agent containing no reducing agent or oxidizing agent. The perm treatment agent into which the metal-doped porous silica surface-modified with a vinylpyrrolidone unit-containing polymer is blended may be in the form of a liquid or cream, for example. The amount of metal-doped porous silica surface-modified with a vinylpyrrolidone unit-containing polymer blended into the perm treatment agent is preferably 0.01 to 5 wt%, and more preferably 0.02 to 0.5 wt%. When the amount of metal-doped porous silica surface-modified with a vinylpyrrolidone unit-containing polymer blended into the perm treatment agent is too small, the deodorizing effect of the metal-doped porous silica on the hair after a perm may be reduced. Meanwhile, when the amount of metal-doped porous silica surface-modified with a vinylpyrrolidone unit-containing polymer blended into the perm treatment agent is too large, the texture of the hair after a perm may deteriorate, or it may take time and effort to wash off the agent. For blending the metal-doped porous silica surface-modified with a vinylpyrrolidone unit-containing polymer into the perm treatment agent, it is possible that a slurry of the metal-doped porous silica surface-modified with a vinylpyrrolidone unit-containing polymer suspended in a dispersion medium is added at any point in the production process of the perm treatment agent, for example.

Incidentally, in the above description, a perm treatment agent has been taken as an example of an article into which the metal-doped porous silica surface-modified with a vinylpyrrolidone unit-containing polymer can be blended. However, the article into which the metal-doped porous silica surface-modified with a vinylpyrrolidone unit-containing polymer can be blended may be any of articles on which the metal-doped porous silica can exert a deodorizing effect, including various cosmetic products, for example, skin care cosmetic products (cosmetic products for cleansing, cosmetic products for skin conditioning, cosmetic products for protection, whitening cosmetic products, UV protection cosmetic products, etc.), makeup cosmetic products (base makeup cosmetic products, point makeup cosmetic products, etc.), hair care cosmetic products (cosmetic products for hair washing, hair styling agents, hair dyes, bleaching agents, etc.), body care cosmetic products (cosmetic products for body washing, bath agents, etc.), and fragrance cosmetic products, as well as quasidrugs such as hair restorers, antiperspirants, and toothpastes.

In addition, a porous silica doped with a metal having antibacterial or antiviral actions, such as copper, can be expected to exert antibacterial or antiviral effects in addition to a deodorizing effect. Therefore, articles into which the porous silica doped with a metal, such as copper, and surface-modified with a vinylpyrrolidone unit-containing polymer can be blended may include liquids and gels for hand disinfection, laundry detergents, laundry softeners, cleaners and cleaning agents (for toilet seat, bathroom, window, etc.), waxes (for floor, wall, etc.), and the like. Further, the porous silica doped with a metal, such as copper, and surface-modified with a vinylpyrrolidone unit-containing polymer may also be blended into articles such as textile products, non-woven fabric products, leather products, building materials, wood materials, paints, adhesives, plastics, films, ceramics, paper, pulp, metalworking oils, water treatment agents, stationeries, toys, containers, caps, pouring tools, and spouts for the purpose of imparting antibacterial or antiviral properties. The method for blending a porous silica doped with a metal, such as copper, and surface-modified with a vinylpyrrolidone unit-containing polymer into such an article may be the same as known methods for blending inorganic antibacterial agents and inorganic antiviral agents.

### Examples

Hereinafter, the present invention will be described in detail with reference to examples. However, the present invention should not be construed as being limited to the following descriptions.

### Production Reference Example 1: Production of Copper- and Aluminum-Doped Mesoporous Silica

Hexadecyltrimethylammonium chloride as a surfactant, copper chloride as a raw material for doping copper in a mesoporous silica, and aluminum chloride as a raw material for doping aluminum in a mesoporous silica were dissolved in water as a solvent, stirred at 100°C for 1 hour, and then cooled to room temperature. After that, tetraethoxysilane as a silica raw material was further dissolved and stirred until uniform. Next, an aqueous sodium hydroxide solution as a basic aqueous solution was added to the reaction liquid such that the pH immediately after the addition was 9, and stirred at room temperature for 20 hours. The formed precipitates were collected by filtration, dried at 50°C for 24 hours, and then calcined at 570°C for 5 hours, thereby giving the intended copper- and aluminum-doped mesoporous silica as a slightly bluish white powder.

Incidentally, the amounts of hexadecyltrimethylammonium chloride as a surfactant, copper chloride as a raw material for doping copper in a mesoporous silica, aluminum chloride as a raw material for doping aluminum in a mesoporous silica, and water as a solvent used per 1 mol of tetraethoxysilane as a silica raw material were each as follows.
Hexadecyltrimethylammonium chloride: 0.225 mol
Copper chloride: 0.0204 mol
Aluminum chloride: 0.0482 mol
Water: 125 mol

In addition, for the preparation of the aqueous sodium hydroxide solution as a basic aqueous solution, 0.195 mol of sodium hydroxide was used per 1 mol of tetraethoxysilane as a silica raw material.

The copper- and aluminum-doped mesoporous silica obtained by the above method had a specific surface area of 1100 m²/g, and the fine pore diameter was about 2.5 nm (calculated by BJH calculation from the adsorption isotherm of nitrogen gas measured at liquid nitrogen temperature by the multi-point method using BELSORP MAX II manufactured by MicrotracBEL Corp.). In addition, about 50 mg of the copper- and aluminum-doped mesoporous silica was accurately weighed out and dissolved in 4 mL of hydrochloric acid, and then the concentrations of copper and aluminum in the hydrochloric acid solution were measured using an Inductively Coupled Plasma-Optical Emission Spectrometer (ICP-OES manufactured by Thermo Scientific). Based on the measurement results, the copper and aluminum contents in the copper- and aluminum-doped mesoporous silica were calculated. As a result, the copper content was 2.09 wt%, and the aluminum content was 2.00 wt%. The doping of the mesoporous silica with copper and aluminum was confirmed using an X-ray photoelectron spectrometer (K-Alpha Surface Analysis manufactured by Thermo Scientific) and a transmission electron microscope (JEM2010 manufactured by JEOL Ltd.).

### Production Reference Example 2: Production of Slurry Containing Copper- and Aluminum-Doped Mesoporous Silica

In a 250 mL I-Boy PP wide-mouth bottle, 11 g of the copper- and aluminum-doped mesoporous silica produced in Production Reference Example 1, 99 g of water, and 220 g of 2 mmφ alumina balls were placed. At room temperature, the bottle was put on a ball mill stand and treated at a rotation speed of 180 rpm for 8 hours, and then the alumina balls were removed, thereby giving a slurry in which the content of a copper- and aluminum-doped mesoporous silica having a median diameter of about 0.5 µm (the median diameter was measured using a laser diffraction particle size distribution analyzer (SALD-3100 manufactured by Shimadzu Corporation) (same hereinafter)) was 10 wt%.

### Production Example 1: Production of Slurry of Copper- and Aluminum-Doped Mesoporous Silica Surface-Modified with Vinylpyrrolidone Unit-Containing Polymer Suspended in Dispersion Medium (No. 1)

In a 250 mL I-Boy PP wide-mouth bottle, 55 g of the slurry obtained in Production Reference Example 2, 11 g of H.C. Polymer 1N (M) from Osaka Organic Chemical Industry Ltd. (containing 20 wt% of polyquaternium-11 having a molecular weight of 500000, Tg: 126°C), 44 g of water, and 220 g of 2 mmφ alumina balls were placed. At room temperature, the bottle was put on a ball mill stand and treated at a rotation speed of 90 rpm for 24 hours, and then the alumina balls were removed, thereby giving a slurry having uniformly dispersed therein a copper- and aluminum-doped mesoporous silica surface-modified with a copolymer of vinylpyrrolidone and dimethylaminoethyl methacrylate (median diameter: about 0.5 µm) (the content of the copper- and aluminum-doped mesoporous silica: 5 wt%, the content of the copolymer of vinylpyrrolidone and dimethylaminoethyl methacrylate: 2 wt%).

### Production Example 2: Production of Slurry of Copper- and Aluminum-Doped Mesoporous Silica Surface-Modified with Vinylpyrrolidone Unit-Containing Polymer Suspended in Dispersion Medium (No. 2)

In a 250 mL I-Boy PP wide-mouth bottle, 55 g of the slurry obtained in Production Reference Example 2, 22 g of a 10 wt% aqueous solution of Polyvinylpyrrolidone K90 from FUJIFILM Wako Pure Chemical Corporation (polyvinylpyrrolidone with undisclosed molecular weight and Tg), 33 g of water, and 220 g of 2 mmφ alumina balls were placed. At room temperature, the bottle was put on a ball mill stand and treated at a rotation speed of 90 rpm for 24 hours, and then the alumina balls were removed, thereby giving a slurry having uniformly dispersed therein a copper- and aluminum-doped mesoporous silica surface-modified with polyvinylpyrrolidone (median diameter: about 0.5 µm) (the content of the copper- and aluminum-doped mesoporous silica: 5 wt%, the content of polyvinylpyrrolidone: 2 wt%).

### Production Example 3: Production of Slurry of Copper- and Aluminum-Doped Mesoporous Silica Surface-Modified with Vinylpyrrolidone Unit-Containing Polymer Suspended in Dispersion Medium (No. 3)

In the same manner as in Production Example 2 except that Polyvinylpyrrolidone K30 from FUJIFILM Wako Pure Chemical Corporation (polyvinylpyrrolidone with undisclosed molecular weight and Tg) was used instead of Polyvinylpyrrolidone K90 from FUJIFILM Wako Pure Chemical Corporation used in Production Example 2, a slurry having uniformly dispersed therein a copper- and aluminum-doped mesoporous silica surface-modified with polyvinylpyrrolidone (median diameter: about 0.5 µm) (the content of the copper- and aluminum-doped mesoporous silica: 5 wt%, the content of polyvinylpyrrolidone: 2 wt%) was obtained.

### Production Example 4: Production of Slurry of Copper- and Aluminum-Doped Mesoporous Silica Surface-Modified with Vinylpyrrolidone Unit-Containing Polymer Suspended in Dispersion Medium (No. 4)

In the same manner as in Production Example 2 except that 11 g of a 10 wt% aqueous solution of Polyvinylpyrrolidone K90 from FUJIFILM Wako Pure Chemical Corporation and 44 g of water were placed in a 250 mL I-Boy PP wide-mouth bottle, a slurry having uniformly dispersed therein a copper- and aluminum-doped mesoporous silica surface-modified with polyvinylpyrrolidone (median diameter: about 0.5 µm) (the content of the copper- and aluminum-doped mesoporous silica: 5 wt%, the content of polyvinylpyrrolidone: 1 wt%) was obtained.

### Production Example 5: Production of Slurry of Copper- and Aluminum-Doped Mesoporous Silica Surface-Modified with Vinylpyrrolidone Unit-Containing Polymer Suspended in Dispersion Medium (No. 5)

In the same manner as in Production Example 2 except that 44 g of a 10 wt% aqueous solution of Polyvinylpyrrolidone K90 from FUJIFILM Wako Pure Chemical Corporation and 11 g of water were placed in a 250 mL I-Boy PP wide-mouth bottle, a slurry having uniformly dispersed therein a copper- and aluminum-doped mesoporous silica surface-modified with polyvinylpyrrolidone (median diameter: about 0.5 µm) (the content of the copper- and aluminum-doped mesoporous silica: 5 wt%, the content of polyvinylpyrrolidone: 4 wt%) was obtained.

### Production Example 6: Production of Slurry of Copper- and Aluminum-Doped Mesoporous Silica Surface-Modified with Vinylpyrrolidone Unit-Containing Polymer Suspended in Dispersion Medium (No. 6)

In the same manner as in Production Example 1 except that H.C. Polymer 1NS from Osaka Organic Chemical Industry Ltd. (containing 20 wt% of polyquaternium-11 having a molecular weight of 500000, Tg: 126°C) was used instead of H.C. Polymer 1N (M) from Osaka Organic Chemical Industry Ltd. used in Production Example 1, a slurry having uniformly dispersed therein a copper- and aluminum-doped mesoporous silica surface-modified with a copolymer of vinylpyrrolidone and dimethylaminoethyl methacrylate (median diameter: about 0.5 µm) (the content of the copper- and aluminum-doped mesoporous silica: 5 wt%, the content of the copolymer of vinylpyrrolidone and dimethylaminoethyl methacrylate: 2 wt%) was obtained.

### Production Example 7: Production of Slurry of Copper- and Aluminum-Doped Mesoporous Silica Surface-Modified with Vinylpyrrolidone Unit-Containing Polymer Suspended in Dispersion Medium (No. 7)

In the same manner as in Production Example 1 except that H.C. Polymer 2L from Osaka Organic Chemical Industry Ltd. (containing 20 wt% of polyquaternium-11 having a molecular weight of 200000, Tg: 126°C) was used instead of H.C. Polymer 1N (M) from Osaka Organic Chemical Industry Ltd. used in Production Example 1, a slurry having uniformly dispersed therein a copper- and aluminum-doped mesoporous silica surface-modified with a copolymer of vinylpyrrolidone and dimethylaminoethyl methacrylate (median diameter: about 0.5 µm) (the content of the copper- and aluminum-doped mesoporous silica: 5 wt%, the content of the copolymer of vinylpyrrolidone and dimethylaminoethyl methacrylate: 2 wt%) was obtained.

### Production Example 8: Production of Slurry of Copper- and Aluminum-Doped Mesoporous Silica Surface-Modified with Vinylpyrrolidone Unit-Containing Polymer Suspended in Dispersion Medium (No. 8)

In the same manner as in Production Example 1 except that H.C. Polymer 3M from Osaka Organic Chemical Industry Ltd. (containing 20 wt% of polyquaternium-11 having a molecular weight of 300000, Tg: 126°C) was used instead of H.C. Polymer 1N (M) from Osaka Organic Chemical Industry Ltd. used in Production Example 1, a slurry having uniformly dispersed therein a copper- and aluminum-doped mesoporous silica surface-modified with a copolymer of vinylpyrrolidone and dimethylaminoethyl methacrylate (median diameter: about 0.5 µm) (the content of the copper- and aluminum-doped mesoporous silica: 5 wt%, the content of the copolymer of vinylpyrrolidone and dimethylaminoethyl methacrylate: 2 wt%) was obtained.

### Production Example 9: Production of Slurry of Copper- and Aluminum-Doped Mesoporous Silica Surface-Modified with Vinylpyrrolidone Unit-Containing Polymer Suspended in Dispersion Medium (No. 9)

In the same manner as in Production Example 1 except that H.C. Polymer 5 from Osaka Organic Chemical Industry Ltd. (containing 20 wt% of polyquaternium-11 having a molecular weight of 150000, Tg: 126°C) was used instead of H.C. Polymer 1N (M) from Osaka Organic Chemical Industry Ltd. used in Production Example 1, a slurry having uniformly dispersed therein a copper- and aluminum-doped mesoporous silica surface-modified with a copolymer of vinylpyrrolidone and dimethylaminoethyl methacrylate (median diameter: about 0.5 µm) (the content of the copper- and aluminum-doped mesoporous silica: 5 wt%, the content of the copolymer of vinylpyrrolidone and dimethylaminoethyl methacrylate: 2 wt%) was obtained.

### Production Example 10: Production of Slurry of Copper- and Aluminum-Doped Mesoporous Silica Surface-Modified with Vinylpyrrolidone Unit-Containing Polymer Suspended in Dispersion Medium (No. 10)

In the same manner as in Production Example 1 except that H.C. Polymer 5W from Osaka Organic Chemical Industry Ltd. (containing 20 wt% of polyquaternium-11 having a molecular weight of 300000, Tg: 126°C) was used instead of H.C. Polymer 1N (M) from Osaka Organic Chemical Industry Ltd. used in Production Example 1, a slurry having uniformly dispersed therein a copper- and aluminum-doped mesoporous silica surface-modified with a copolymer of vinylpyrrolidone and dimethylaminoethyl methacrylate (median diameter: about 0.5 µm) (the content of the copper- and aluminum-doped mesoporous silica: 5 wt%, the content of the copolymer of vinylpyrrolidone and dimethylaminoethyl methacrylate: 2 wt%) was obtained.

### Production Example 11: Production of Slurry of Copper- and Aluminum-Doped Mesoporous Silica Surface-Modified with Vinylpyrrolidone Unit-Containing Polymer Suspended in Dispersion Medium (No. 11)

In the same manner as in Production Example 2 except that Luviskol K90 from BASF Japan Ltd. (polyvinylpyrrolidone having a molecular weight of 1200000, Tg: undisclosed) was used instead of Polyvinylpyrrolidone K90 from FUJIFILM Wako Pure Chemical Corporation used in Production Example 2, a slurry having uniformly dispersed therein a copper- and aluminum-doped mesoporous silica surface-modified with polyvinylpyrrolidone (median diameter: about 0.5 µm) (the content of the copper- and aluminum-doped mesoporous silica: 5 wt%, the content of polyvinylpyrrolidone: 2 wt%) was obtained.

### Production Example 12: Production of Slurry of Copper- and Aluminum-Doped Mesoporous Silica Surface-Modified with Vinylpyrrolidone Unit-Containing Polymer Suspended in Dispersion Medium (No. 12)

In the same manner as in Production Example 2 except that CREEJUS K-90 from DKS Co. Ltd. (polyvinylpyrrolidone having a molecular weight of 1200000, Tg: undisclosed) was used instead of Polyvinylpyrrolidone K90 from FUJIFILM Wako Pure Chemical Corporation used in Production Example 2, a slurry having uniformly dispersed therein a copper- and aluminum-doped mesoporous silica surface-modified with polyvinylpyrrolidone (median diameter: about 0.5 µm) (the content of the copper- and aluminum-doped mesoporous silica: 5 wt%, the content of polyvinylpyrrolidone: 2 wt%) was obtained.

### Production Example 13: Production of Slurry of Copper- and Aluminum-Doped Mesoporous Silica Surface-Modified with Vinylpyrrolidone Unit-Containing Polymer Suspended in Dispersion Medium (No. 13)

In the same manner as in Production Example 2 except that PVP K-90 from Ashland Inc. (polyvinylpyrrolidone having a molecular weight of 900000, Tg: undisclosed) was used instead of Polyvinylpyrrolidone K90 from FUJIFILM Wako Pure Chemical Corporation used in Production Example 2, a slurry having uniformly dispersed therein a copper- and aluminum-doped mesoporous silica surface-modified with polyvinylpyrrolidone (median diameter: about 0.5 µm) (the content of the copper- and aluminum-doped mesoporous silica: 5 wt%, the content of polyvinylpyrrolidone: 2 wt%) was obtained.

### Production Example 14: Production of Slurry of Copper- and Aluminum-Doped Mesoporous Silica Surface-Modified with Vinylpyrrolidone Unit-Containing Polymer Suspended in Dispersion Medium (No. 14)

In the same manner as in Production Example 1 except that Copolymer 845 from Ashland Inc. (containing 20 wt% of a copolymer of vinylpyrrolidone and dimethylaminoethyl methacrylate having a molecular weight of 1000000, Tg: 172°C) was used instead of H.C. Polymer 1N (M) from Osaka Organic Chemical Industry Ltd. used in Production Example 1, a slurry having uniformly dispersed therein a copper- and aluminum-doped mesoporous silica surface-modified with a copolymer of vinylpyrrolidone and dimethylaminoethyl methacrylate (median diameter: about 0.5 µm) (the content of the copper- and aluminum-doped mesoporous silica: 5 wt%, the content of the copolymer of vinylpyrrolidone and dimethylaminoethyl methacrylate: 2 wt%) was obtained.

### Production Example 15: Production of Slurry of Copper- and Aluminum-Doped Mesoporous Silica Suspended in Dispersion Medium

At room temperature, 50 g of water was added to 50 g of the slurry obtained in Production Reference Example 2, thereby giving a slurry having uniformly dispersed therein a copper- and aluminum-doped mesoporous silica (median diameter: about 0.5 µm) (the content of the copper- and aluminum-doped mesoporous silica: 5 wt%).

### Production Example 16: Production of Slurry of Copper- and Aluminum-Doped Mesoporous Silica Surface-Modified with Dodecylamine Suspended in Dispersion Medium

In a 250 mL I-Boy PP wide-mouth bottle, 50 g of the slurry obtained in Production Reference Example 2, 1 g of dodecylamine hydrochloride from Tokyo Chemical Industry Co., Ltd., and 49 g of water were placed and, at room temperature, thoroughly shaken and stirred, thereby giving a slurry having uniformly dispersed therein a copper- and aluminum-doped mesoporous silica surface-modified with dodecylamine (median diameter: about 0.5 µm) (the content of the copper- and aluminum-doped mesoporous silica: 5 wt%, the content of dodecylamine: 1 wt%).

### Production Example 17: Production of Slurry of Copper- and Aluminum-Doped Mesoporous Silica Surface-Modified with Mixture of High-Molecular-Weight Block Copolymer and TWEEN^{®}-20 Suspended in Dispersion Medium

In a 250 mL I-Boy PP wide-mouth bottle, 50 g of the slurry obtained in Production Reference Example 2, 0.25 g of DISPERBYK-190 from BYK Japan KK (containing 40 wt% of a high-molecular-weight block copolymer), 0.25 g of TWEEN^{®}-20 from FUJIFILM Wako Pure Chemical Corporation, and 49.5 g of water were placed and, at room temperature, thoroughly shaken and stirred, thereby giving a slurry having uniformly dispersed therein a copper- and aluminum-doped mesoporous silica surface-modified with a mixture of a high-molecular-weight block copolymer and TWEEN^{®}-20 (median diameter: about 0.5 µm) (the content of the copper- and aluminum-doped mesoporous silica: 5 wt%, the content of the high-molecular-weight block copolymer: 0.10 wt%, the content of TWEEN^{®}-20: 0.25 wt%).

### Production Example 18: Production of Slurry of Copper- and Aluminum-Doped Mesoporous Silica Surface-Modified with Silicone Polymer Terminally Modified with Amino Group (Amodimethicone) Suspended in Dispersion Medium

In the same manner as in Production Example 2 except that a 10 wt% aqueous solution of amodimethicone prepared by diluting DOWSIL FZ-4671 from Dow Toray Co., Ltd. (containing 31.7 wt% of amodimethicone) with water was used instead of the 10 wt% aqueous solution of Polyvinylpyrrolidone K90 from FUJIFILM Wako Pure Chemical Corporation used in Production Example 2, an attempt was made to give a slurry having uniformly dispersed therein a copper- and aluminum-doped mesoporous silica surface-modified with amodimethicone (median diameter: about 0.5 µm) (the content of the copper- and aluminum-doped mesoporous silica: 5 wt%, the content of amodimethicone: 2 wt%) . However, a purple, sticky, foamy viscous liquid stuck to the inner wall of the bottle and the alumina ball surface, and it was not possible to obtain such a slurry. This was presumably because a plurality of amino groups in amodimethicone caused cross-linking of particles of the copper- and aluminum-doped mesoporous silica, resulting in aggregation and agglomeration.

The slurries produced in Production Examples 1 to 18 are summarized in Table 1.

**[Table 1]**

| Slurry | Metal-Doped Silica | | Surface Modifier | | | | Surface Modifier/Metal -Doped Silica | Metal-Doped Silica Median Diameter |
|---|---|---|---|---|---|---|---|---|
| | Kind | Content in Slurry | Kind | Molecular Weight | Tg | AmountAdded | | |
| Slurry of Production Example 1 | Cu, Al-doped mesoporous silica | 5.0 wt% | Polyquaternium-11 | 500000 | 126°C | 2.0 wt% | 40% | About 0.5 µm |
| Slurry of Production Example 2 | Cu, Al-doped mesoporous silica | 5.0 wt% | Polyvinylpyrrolidone K90 | Unknown | Unknown | 2.0 wt% | 40% | About 0.5 µm |
| Slurry of Production Example 3 | Cu, Al-doped mesoporous silica | 5.0 wt% | Polyvinylpyrrolidone K30 | Unknown | Unknown | 2.0 wt% | 40% | About 0.5 µm |
| Slurry of Production Example 4 | Cu, Al-doped mesoporous silica | 5.0 wt% | Polyvinylpyrrolidone K90 | Unknown | Unknown | 1.0 wt% | 20% | About 0.5 µm |
| Slurry of Production Example 5 | Cu, Al-doped mesoporous silica | 5.0 wt% | Polyvinylpyrrolidone K90 | Unknown | Unknown | 4.0 wt% | 80% | About 0.5 µm |
| Slurry of Production Example 6 | Cu, Al-doped mesoporous silica | 5.0 wt% | Polyquaternium-11 | 500000 | 126°C | 2.0 wt% | 40% | About 0.5 µm |
| Slurry of Production Example 7 | Cu, Al-doped mesoporous silica | 5.0 wt% | Polyquaternium-11 | 200000 | 126°C | 2.0 wt% | 40% | About 0.5 µm |
| Slurry of Production Example 8 | Cu, Al-doped mesoporous silica | 5.0 wt% | Polyquaternium-11 | 300000 | 126°C | 2.0 wt% | 40% | About 0.5 µm |
| Slurry of Production Example 9 | Cu, Al-doped mesoporous silica | 5.0 wt% | Polyquaternium-11 | 150000 | 126°C | 2.0 wt% | 40% | About 0.5 µm |
| Slurry of Production Example 10 | Cu, Al-doped mesoporous silica | 5.0 wt% | Polyquaternium-11 | 300000 | 126°C | 2.0 wt% | 40% | About 0.5 µm |
| Slurry of Production Example 11 | Cu, Al-doped mesoporous silica | 5.0 wt% | Polyvinylpyrrolidone K90 | 1200000 | Unknown | 2.0 wt% | 40% | About 0.5 µm |
| Slurry of Production Example 12 | Cu, Al-doped mesoporous silica | 5.0 wt% | Polyvinylpyrrolidone K90 | 1200000 | Unknown | 2.0 wt% | 40% | About 0.5 µm |
| Slurry of Production Example 13 | Cu, Al-doped mesoporous silica | 5.0 wt% | Polyvinylpyrrolidone K90 | 900000 | Unknown | 2.0 wt% | 40% | About 0.5 µm |
| Slurry of Production Example 14 | Cu, Al-doped mesoporous silica | 5.0 wt% | Vinyl pyrrolidone/ dimethylaminoethyl methacrylate copolymer | 1000000 | 172°C | 2.0 wt% | 40% | About 0.5 µm |
| Slurry of Production Example 15 | Cu, Al-doped mesoporous silica | 5.0 wt% | - | - | - | - | - | About 0.5 µm |
| Slurry of Production Example 16 | Cu, Al-doped mesoporous silica | 5.0 wt% | Dodecylamine hydrochloride | 221.81 | - | 1.0 wt% | 20% | About 0.5 µm |
| Slurry of Production Example 17 | Cu, Al-doped mesoporous silica | 5.0 wt% | DISPERBYK-190 TWEEN-20 | Unknown | Unknown | 0.35 wt% in total | 7% in total | About 0.5 µm |
| Slurry of Production Example 18 (Faulty Production) | Cu, Al-doped mesoporous silica | 5.0 wt% | Amodimethicone | Unknown | Unknown | 2.0 wt% | 40% | About 0.5 µm |

| | | | | | | | | |
|---|---|---|---|---|---|---|---|---|
| * "Unknown" for Molecular Weight and Tg means no information disclosure from the source | | | | | | | | |

### Reference Example 1: Analysis of Copper- and Aluminum-Doped Mesoporous Silica Surface-Modified with Polyvinylpyrrolidone Contained in Slurry Produced in Production Examples 2, 4, and 5

The slurries produced in Production Examples 2, 4, and 5 were each suction-filtered through a 70 mmφ filter paper, and the copper- and aluminum-doped mesoporous silica surface-modified with polyvinylpyrrolidone was collected on the filter paper. The collected copper- and aluminum-doped mesoporous silica surface-modified with polyvinylpyrrolidone was, without washing with water, dried at 100°C for about 1 hour and cooled. Subsequently, about 8 mg thereof was weighed out, heated from 40°C to 600°C at a heating rate of 5°C/min, and held at 600°C for 1 hour, and the mass change at the holding time was measured using a thermal analyzer (STA7220 manufactured by Hitachi High-Tech Science Corporation). Supposing that water contained in the copper- and aluminum-doped mesoporous silica surface-modified with polyvinylpyrrolidone evaporates up to 100°C, and polyvinylpyrrolidone adhering to the copper- and aluminum-doped mesoporous silica disappears after 100°C, from the calculation formula ((A - B)/A) × 100 (A: weight before heating - weight loss up to 100°C, B: weight after heating), the proportion of the weight of polyvinylpyrrolidone in the weight of the copper- and aluminum-doped mesoporous silica surface-modified with polyvinylpyrrolidone (measured value: %) was calculated. In addition, from the weight of the copper- and aluminum-doped mesoporous silica and the weight of polyvinylpyrrolidone used in the slurry production, the proportion of the weight of polyvinylpyrrolidone based on their total weight (calculated value: %) was calculated. The measured and calculated values are shown in Table 2.

**[Table 2]**

| Slurry | Metal-Doped Silica | | Surface Modifier | | Proportion of Surface Modifier | |
|---|---|---|---|---|---|---|
| | Kind | Content in Slurry | Kind | Amount Added | Measured Value | Calculated Value |
| Slurry of Production Example 4 | Cu, Al-doped mesoporous silica | 5.0 wt% | Polyvinylpyrrolidone K90 | 1.0 wt% | 18% | 17% |
| Slurry of Production Example 2 | Cu, Al-doped mesoporous silica | 5.0 wt% | Polyvinylpyrrolidone K90 | 2.0 wt% | 28% | 29% |
| Slurry of Production Example 5 | Cu, Al-doped mesoporous silica | 5.0 wt% | Polyvinylpyrrolidone K90 | 4.0 wt% | 36% | 44% |

As is clear from Table 2, in the slurries produced in Production Examples 4 and 2, the proportion of polyvinylpyrrolidone is almost the same between the measured and calculated values. From this, it turned out that at least when the weight of polyvinylpyrrolidone used in the slurry production is up to 0.4 times the weight of the copper- and aluminum-doped mesoporous silica used, the entire amount of polyvinylpyrrolidone adheres to the copper- and aluminum-doped mesoporous silica. On the other hand, in the slurry produced in Production Example 5, the measured value is smaller than the calculated value. From this, it turned out that not the entire amount of polyvinylpyrrolidone used in the slurry production adhered to the copper- and aluminum-doped mesoporous silica, and free polyvinylpyrrolidone was contained in the slurry.

### Test Example 1: Evaluation of Dispersibility in Aqueous Solution of Polyquaternium-10

### (Evaluation Method)

0.5 mL of each of the slurries produced in Production Examples 2 and 15 and 1.5 mL of water were added to 8 mL of a 1.25 wt% aqueous solution of polyquaternium-10 (manufactured by Sigma-Aldrich Co. LLC) placed in a glass container, stirred by thorough shaking at room temperature for 10 seconds, and then allowed to stand for 60 minutes. The appearance of the resulting mixture was visually observed, and rated as "o" when the copper- and aluminum-doped mesoporous silica was kept stably dispersed, or as "×" when not kept dispersed, forming precipitates.

### (Evaluation Results)

The results are shown in Table 3. As is clear from Table 3, in the visual evaluation of the appearance of the mixture, use of the slurry produced in Production Example 2 resulted in "∘", while use of the slurry produced in Production Example 15 resulted in "×". Thus, it turned out that when a copper- and aluminum-doped mesoporous silica is surface-modified with polyvinylpyrrolidone, and blended into an aqueous solution of polyquaternium-10, the copper- and aluminum-doped mesoporous silica is kept stably dispersed.

### Test Example 2: Evaluation of Dispersibility in Aqueous Solution of Polyquaternium-11

### (Evaluation Method)

0.5 mL of each of the slurries produced in Production Examples 1 to 17 and 1.5 mL of water were added to 8 mL of a 1.25 wt% aqueous solution of polyquaternium-11 (prepared using H.C. Polymer 1N (M) from Osaka Organic Chemical Industry Ltd.) placed in a glass container, stirred by thorough shaking at room temperature for 10 seconds, and then allowed to stand for 60 minutes. The appearance of the resulting mixture was visually observed, and rated as "o" when the copper- and aluminum-doped mesoporous silica was kept stably dispersed, or as "×" when not kept dispersed, forming precipitates.

### (Evaluation Results)

The results are shown in Table 3. As is clear from Table 3, in the visual evaluation of the appearance of the mixture, use of the slurries produced in Production Examples 1 to 14 all resulted in "o", while use of the slurries produced in Production Examples 15 to 17 all resulted in "×". Thus, it turned out that when a copper- and aluminum-doped mesoporous silica is surface-modified with a copolymer of vinylpyrrolidone and dimethylaminoethyl methacrylate, or polyvinylpyrrolidone, and blended into an aqueous solution of polyquaternium-11, the copper- and aluminum-doped mesoporous silica is kept stably dispersed.

### Test Example 3: Evaluation of Dispersibility in Aqueous Dispersion of Amodimethicone

### (Evaluation Method)

0.5 mL of each of the slurries produced in Production Examples 1 to 17 and 1.5 mL of water were added to 8 mL of a 1.25 wt% aqueous solution of amodimethicone (prepared using DOWSIL FZ-4671 from Dow Toray Co., Ltd.) placed in a glass container, stirred by thorough shaking at room temperature for 10 seconds, and then allowed to stand for 60 minutes. The appearance of the resulting mixture was visually observed, and rated as "o" when the copper- and aluminum-doped mesoporous silica was kept stably dispersed, or as "×" when not kept dispersed, forming precipitates.

### (Evaluation Results)

The results are shown in Table 3. As is clear from Table 3, in the visual evaluation of the appearance of the mixture, use of the slurries produced in Production Examples 1 to 14 all resulted in "o", while use of the slurries produced in Production Examples 15 to 17 all resulted in "×". Thus, it turned out that when a copper- and aluminum-doped mesoporous silica is surface-modified with a copolymer of vinylpyrrolidone and dimethylaminoethyl methacrylate, or polyvinylpyrrolidone, and blended into an aqueous dispersion of amodimethicone, the copper- and aluminum-doped mesoporous silica is kept stably dispersed.

### Test Example 4: Evaluation of Dispersibility in Aqueous Solution of Polyvinylpyrrolidone

### (Evaluation Method)

0.5 mL of each of the slurries produced in Production Examples 2 and 15 and 1.5 mL of water were added to 8 mL of a 1.25 %wt aqueous solution of polyvinylpyrrolidone (prepared using Luviskol K90 from BASF Japan Ltd.) placed in a glass container, stirred by thorough shaking at room temperature for 10 seconds, and then allowed to stand for 60 minutes. The appearance of the resulting mixture was visually observed, and rated as "o" when the copper- and aluminum-doped mesoporous silica was kept stably dispersed, or as "×" when not kept dispersed, forming precipitates.

### (Evaluation Results)

The results are shown in Table 3. As is clear from Table 3, in the visual evaluation of the appearance of the mixture, use of the slurry produced in Production Example 2 resulted in "o", while use of the slurry produced in Production Example 15 resulted in "×". Thus, it turned out that when a copper- and aluminum-doped mesoporous silica is surface-modified with polyvinylpyrrolidone, and blended into an aqueous solution of polyvinylpyrrolidone, the copper- and aluminum-doped mesoporous silica is kept stably dispersed.

### Test Example 5: Evaluation of Adsorption Action on Cysteamine

### (Evaluation Method)

1.8 mL of water was added to a centrifuge tube containing 0.1 mL of each of the slurries produced in Production Examples 1 to 17, and thoroughly shaken at room temperature to make a uniform dispersion. Subsequently, 0.1 mL of an aqueous cysteamine solution having a concentration of 5.86 wt% was further added, thoroughly shaken for 30 seconds, and then centrifuged for 90 seconds. After that, the supernatant was taken out from the centrifuge tube and measured for absorbance at 235 nm. From the calibration curve of the concentration of the aqueous cysteamine solution and the absorbance, the cysteamine concentration of the supernatant was determined, and, from the calculation formula ((0.293 wt% - cysteamine concentration of supernatant)/0.293 wt%) × 100, the adsorption rate (%) of each of the slurries produced in Production Examples 1 to 17 on cysteamine was calculated. Incidentally, the absorbance was measured using a Corona Grating Microplate Reader SH-1000 from Corona Electric Co., Ltd.

### (Evaluation Results)

The results are shown in Table 3. As is clear from Table 3, all the slurries produced in Production Examples 1 to 17 had high adsorption on cysteamine, and no decrease in the adsorption on cysteamine due to the surface modification of the copper- and aluminum-doped mesoporous silica with a surface modifier was observed.

### Reference Example 2: Zeta Potential of Slurry Produced in Production Examples 1 to 17

Measurement was performed in a zeta potential/particle size/molecular weight measurement system (ELSZ-2000ZS) from Otsuka Electronics Co., Ltd. The results are shown in Table 3. In general, the greater the absolute value of the zeta potential, the greater the electrostatic repulsive force and the higher the dispersion stability. In fact, the slurry produced in Production Example 15 is a slurry having uniformly dispersed therein a copper- and aluminum-doped mesoporous silica, and the absolute value of the zeta potential is 30 mV or more. However, even in the case of such a slurry having uniformly dispersed therein a copper- and aluminum-doped mesoporous silica, when blended into an aqueous solution or aqueous dispersion of a cationic polymer such as polyquaternium-10, polyquaternium-11, or amodimethicone, charge compensation occurs between the negatively charged copper- and aluminum-doped mesoporous silica and the cationic polymer, and, as a result, cross-linking occurs due to the adsorption of the two, etc., resulting in aggregation and agglomeration, causing precipitation. In contrast, with respect to the slurries produced in Production Examples 1 to 14, although the absolute value of the zeta potential of each slurry is smaller than the absolute value of the zeta potential of the slurry produced in Production Example 15, and the electrostatic repulsive force is smaller, the dispersion stability in the slurry is high. This is presumably attributable to the repulsive force due to the high steric hindrance of the vinylpyrrolidone unit-containing polymer present on the surface of the copper- and aluminum-doped mesoporous silica, and it is considered that even after the slurry is blended into an aqueous solution or aqueous dispersion of a cationic polymer, this repulsive force inhibits agglomeration and agglomeration with the cationic polymer, contributing to the maintenance of dispersion stability. The reason why the slurries produced in Production Examples 16 and 17 cannot maintain dispersion stability after being blended into an aqueous solution or aqueous dispersion of a cationic polymer is presumably because unlike a vinylpyrrolidone unit-containing polymer, the surface modifier used has a chemical structure that does not bring about repulsive force due to high steric hindrance. The reason why precipitation occurs as a result of blending the slurry produced in Production Example 15 into an aqueous solution of a nonionic polymer polyvinylpyrrolidone is presumably not because of the charge compensation as described above, and is not necessarily clear. However, the reason why no precipitation occurs as a result of blending the slurry produced in Production Example 2 is presumably attributable, again, to the repulsive force due to the high steric hindrance of polyvinylpyrrolidone present on the surface of the copper- and aluminum-doped mesoporous silica.

**[Table 3]**

| | Slurry | Surface Modifier | | Dispersibility in Cosmetic Product Raw Material Polymer Liquid | | | | Cysteamine Adsorption Rate | Zeta Potential (mV) |
|---|---|---|---|---|---|---|---|---|---|
| | | Kind | Amount Added | Polyquaternium-10 Liquid | Polyquaternium-11 Liquid | Amodimethicone Liquid | Polyvinylpyrrolidone Liquid | | |
| | Slurry of Production Example 1 | Polyquaternium-11 | 2.0 wt% | Not performed | ○ | ○ | Not performed | 96.0% | 4.25 |
| | Slurry of Production Example 2 | Polyvinylpyrrolidone K90 | 2.0 wt% | ○ | ○ | ○ | ○ | 96.3% | -2.69 |
| | Slurry of Production Example 3 | Polyvinylpyrrolidone K30 | 2.0 wt% | Not performed | ○ | ○ | Not performed | 96.0% | -11.07 |
| | Slurry of Production Example 4 | Polyvinylpyrrolidone K90 | 1.0 wt% | Not performed | ○ | ○ | Not performed | 96.2% | -4.35 |
| | Slurry of Production Example 5 | Polyvinylpyrrolidone K90 | 4.0 wt% | Not performed | ○ | ○ | Not performed | 96.1 % | 0.69 |
| | Slurry of Production Example 6 | Polyquaternium-11 | 2.0 wt% | Not performed | ○ | ○ | Not performed | 96.0% | 15.37 |
| | Slurry of Production Example 7 | Polyquaternium-11 | 2.0 wt% | Not performed | ○ | ○ | Not performed | 96.0% | 1.42 |
| Examples | Slurry of Production Example 8 | Polyquaternium-11 | 2.0 wt% | Not performed | ○ | ○ | Not performed | 96.0% | 31.12 |
| | Slurry of Production Example 9 | Polyquaternium-11 | 2.0 wt% | Not performed | ○ | ○ | Not performed | 96.0% | 2.69 |
| | Slurry of Production Example 10 | Polyquaternium-11 | 2.0 wt% | Not performed | ○ | ○ | Not performed | 96.0% | 1.35 |
| | Slurry of Production Example 11 | Polyvinylpyrrolidone K90 | 2.0 wt% | Not performed | ○ | ○ | Not performed | 96.0% | -1.07 |
| | Slurry of Production Example 12 | Polyvinylpyrrolidone K90 | 2.0 wt% | Not performed | ○ | ○ | Not performed | 96.0% | 0.30 |
| | Slurry of Production Example 13 | Polyvinylpyrrolidone K90 | 2.0 wt% | Not performed | ○ | ○ | Not performed | 96.0% | 0.70 |
| | Slurry of Production Example 14 | Vinyl pyrrolidone/ dimethylaminoethyl methacrylate copolymer | 2.0 wt% | Not performed | ○ | ○ | Not performed | 96.0% | 1.13 |
| Comparative Examples | Slurry of Production Example 15 | - | - | × | × | × | × | 96.0% | -34.73 |
| | Slurry of Production Example 16 | Dodecylamine hydrochloride | 1.0 wt% | Not performed | × | × | Not performed | 95.7% | -4.32 |
| | Slurry of Production Example 17 | DISPERBYK-190 TWEEN-20 | 0.35 wt%in total | Not performed | × | × | Not performed | 96.1% | -15.38 |
| | Slurry of Production Example 18 (Faulty Production) | Amodimethicone | 2.0 wt% | Not performed | Not performed | Not performed | Not performed | Not performed | Not performed |

### Application Example 1: Production of Perm Treatment Agent Having Blended Therein Metal-Doped Porous Silica Surface-Modified with Vinylpyrrolidone Unit-Containing Polymer

The slurry containing a copper- and aluminum-doped mesoporous silica surface-modified with a copolymer of vinylpyrrolidone and dimethylaminoethyl methacrylate obtained in Production Example 1 was added to a commercially available perm treatment agent (second agent) containing at least polyquaternium-11, and thoroughly stirred at room temperature. As a result, it was possible to produce a perm treatment agent having uniformly dispersed therein, at a content of 0.5 wt%, a copper- and aluminum-doped mesoporous silica surface-modified with a copolymer of vinylpyrrolidone and dimethylaminoethyl methacrylate.

### Application Example 2: Production of Shampoo Agent Having Blended Therein Metal-Doped Porous Silica Surface-Modified with Vinylpyrrolidone Unit-Containing Polymer

The slurry containing a copper- and aluminum-doped mesoporous silica surface-modified with polyvinylpyrrolidone obtained in Production Example 2 was added to a commercially available shampoo agent containing at least polyquaternium-10, and thoroughly stirred at room temperature. As a result, it was possible to produce a shampoo agent having uniformly dispersed therein, at a content of 0.5 wt%, a copper- and aluminum-doped mesoporous silica surface-modified with polyvinylpyrrolidone.

### Application Example 3: Production of Hair Treatment Agent Having Blended Therein Metal-Doped Porous Silica Surface-Modified with Vinylpyrrolidone Unit-Containing Polymer

The slurry containing a copper- and aluminum-doped mesoporous silica surface-modified with polyvinylpyrrolidone obtained in Production Example 2 was added to a commercially available hair treatment agent containing at least amodimethicone, and thoroughly stirred at room temperature. As a result, it was possible to produce a hair treatment agent having uniformly dispersed therein, at a content of 0.5 wt%, a copper- and aluminum-doped mesoporous silica surface-modified with polyvinylpyrrolidone.

### Application Example 4: Production of Hair Styling Agent Having Blended Therein Metal-Doped Porous Silica Surface-Modified with Vinylpyrrolidone Unit-Containing Polymer

The slurry containing a copper- and aluminum-doped mesoporous silica surface-modified with polyvinylpyrrolidone obtained in Production Example 2 was added to a commercially available hair styling agent containing at least polyvinylpyrrolidone, and thoroughly stirred at room temperature. As a result, it was possible to produce a hair styling agent having uniformly dispersed therein, at a content of 0.5 wt%, a copper- and aluminum-doped mesoporous silica surface-modified with polyvinylpyrrolidone.

### Application Example 5: Production of Toilet Seat Cleaner Having Blended Therein Metal-Doped Porous Silica Surface-Modified with Vinylpyrrolidone Unit-Containing Polymer

The slurry containing a copper- and aluminum-doped mesoporous silica surface-modified with polyvinylpyrrolidone obtained in Production Example 2 was added to a commercially available toilet seat cleaner containing at least polyquaternium-55, and thoroughly stirred at room temperature. As a result, it was possible to produce a toilet seat cleaner having uniformly dispersed therein, at a content of 0.5 wt%, a copper- and aluminum-doped mesoporous silica surface-modified with polyvinylpyrrolidone.

### Application Example 6: Production of Alcoholic Hand Gel Having Blended Therein Metal-Doped Porous Silica Surface-Modified with Vinylpyrrolidone Unit-Containing Polymer

The slurry containing a copper- and aluminum-doped mesoporous silica surface-modified with polyvinylpyrrolidone obtained in Production Example 13 was added to a commercially available alcoholic hand gel containing at least carbomer, and thoroughly stirred at room temperature. As a result, it was possible to produce an alcoholic hand gel having uniformly dispersed therein, at a content of 0.5 wt%, a copper- and aluminum-doped mesoporous silica surface-modified with polyvinylpyrrolidone.

### Industrial Applicability

The present invention makes it possible to provide a metal-doped porous silica that can be blended into an article, for example, a cosmetic product such as a perm treatment agent, and kept stably dispersed therein. In this respect, the present invention is industrially applicable.

## Claims

1. A metal-doped porous silica surface-modified with a vinylpyrrolidone unit-containing polymer.

2. The metal-doped porous silica according to claim 1, wherein the metal doped in the porous silica is at least one member selected from the group consisting of copper, aluminum, zirconium, cobalt, manganese, and iron.

3. The metal-doped porous silica according to claim 2, wherein the metal doped in the porous silica is copper and/or aluminum.

4. The metal-doped porous silica according to claim 1, wherein the vinylpyrrolidone unit-containing polymer is a copolymer of a vinylpyrrolidone unit and a non-vinylpyrrolidone unit.

5. The metal-doped porous silica according to claim 4, wherein the copolymer of a vinylpyrrolidone unit and a non-vinylpyrrolidone unit is a copolymer of vinylpyrrolidone and dimethylaminoethyl methacrylate.

6. The metal-doped porous silica according to claim 1, wherein the vinylpyrrolidone unit-containing polymer is polyvinylpyrrolidone.

7. A method for producing a metal-doped porous silica surface-modified with a vinylpyrrolidone unit-containing polymer, comprising a step in which a slurry of a metal-doped porous silica suspended in a dispersion medium is housed in a treatment container together with a vinylpyrrolidone unit-containing polymer and balls for use in ball milling (media) (a dispersion medium may further be housed), and the treatment container housing them is put on a ball mill stand and rotated, thereby surface-treating the metal-doped porous silica.

8. A slurry comprising a metal-doped porous silica surface-modified with a vinylpyrrolidone unit-containing polymer suspended in a dispersion medium.

9. Use of the metal-doped porous silica surface-modified with a vinylpyrrolidone unit-containing polymer according to claim 1 for deodorizing an article by blending into an article containing at least one member selected from the group consisting of polyquaternium-10, polyquaternium-11, amodimethicone, and polyvinylpyrrolidone.
